**Europäisches Patentamt**

**European Patent Office**

**Office européen des brevets**

(19)

(11) Veröffentlichungsnummer: **0 356 395**
**A2**

(12) # EUROPÄISCHE PATENTANMELDUNG

(21) Anmeldenummer: **89810612.5**

(22) Anmeldetag: **17.08.89**

(51) Int. Cl.⁵: **C 07 K 7/10**
**A 61 K 37/02, C 12 N 15/00,**
**C 07 K 1/00**

(30) Priorität: **26.08.88 CH 3174/88**

(43) Veröffentlichungstag der Anmeldung:
**28.02.90 Patentblatt 90/09**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Rink, Hans**
**Rebenstrasse 10**
**CH-4125 Riehen (CH)**

**Müller, Klaus, Dr.**
**Fürstensteinhof 17**
**CH-4107 Ettingen (CH)**

Patentansprüche für folgende Vertragsstaaten: ES + GR.

(54) **Flankierende Peptide und Verfahren zu ihrer Herstellung.**

(57) Peptide der Formel I,

```
R-Ala-Pro-Phe-Arg-Ser-Ala-Leu-Glu-Ser-Ser-
  Pro-Ala-Asp-Pro-Ala-Thr-Leu-Ser-Glu-Asp-
  Glu-Ala-Arg-Leu-Leu-Leu-Ala-Ala-Leu-Val-          (I)
  Gln-Asp-Tyr-Val-Gln-Met-Lys-Ala-Ser-Glu-
  Leu-Glu-Gln-Glu-Gln-Glu-Arg-Glu-Gly-Ser-X-OH
```

worin R Wasserstoff oder Acetyl und X die Aminosäuresequenz der Formel -Ser-Leu-Asp-Ser-Pro-Arg-Ser- (Ia) oder der Formel -Arg-Ile-Ile-Ala-Gln- (Ib) bedeuten, und Salze von solchen Verbindungen können zur Behandlung von Erkrankungen mit Osteopenien verwendet werden.

EP 0 356 395 A2

**Beschreibung**

### Flankierende Peptide und Verfahren zu ihrer Herstellung

Die Erfindung betrifft neue Peptide, insbesondere Humanprocalcitonin N-terminal-flankierende Peptide, deren Salze, gewisse DNA-Sequenzen und Mikroorganismen, die solche DNA-Sequenzen enthalten und zur Produktion der erfindungsgemässen Peptide fähig sind, als Zwischenprodukte zur Herstellung dieser Peptide, Verfahren zur Herstellung der Peptide oder DNA-Sequenzen, pharmazeutische Präparate, die die genannten Peptide oder Salze davon enthalten, sowie die Verwendung dieser Peptide oder ihrer Salze als Arzneimittel.

Die Erfindung betrifft insbesondere Peptide der Formel I,

R—Ala—Pro—Phe—Arg—Ser—Ala—Leu—Glu—Ser—Ser—

Pro—Ala—Asp—Pro—Ala—Thr—Leu—Ser—Glu—Asp—

Glu—Ala—Arg—Leu—Leu—Leu—Ala—Ala—Leu—Val—       (I)

Gln—Asp—Tyr—Val—Gln—Met—Lys—Ala—Ser—Glu—

Leu—Glu—Gln—Glu—Gln—Glu—Arg—Glu—Gly—Ser—X—OH

worin R Wasserstoff oder Acetyl und X die Aminosäuresequenz der Formel -Ser-Leu-Asp-Ser-Pro-Arg-Ser- (Ia) oder der Formel -Arg-Ile-Ile-Ala-Gln- (Ib) bedeuten, aufweisen, insbesondere die Peptide der Formel

H—Ala—Pro—Phe—Arg—Ser—Ala—Leu—Glu—Ser—Ser—

Pro—Ala—Asp—Pro—Ala—Thr—Leu—Ser—Glu—Asp—

Glu—Ala—Arg—Leu—Leu—Leu—Ala—Ala—Leu—Val—       (II)

Gln—Asp—Tyr—Val—Gln—Met—Lys—Ala—Ser—Glu—

Leu—Glu—Gln—Glu—Gln—Glu—Arg—Glu—Gly—Ser—X—OH

worin X vorzugsweise für die Aminosäuresequenz der Formel Ia (PCATFP: human procalcitonin amino-terminal flanking peptide), die auch als PAS-57 (Peptid Alanin Serin-57) bezeichnet wird, ferner für diejenige der Formel Ib (PCGRPATFP: human procalcitonin gene related peptide amino-terminal flanking peptide) steht, sowie Salze von solchen Peptiden.

In Uebereinstimmung mit den international anerkannten Nomenklaturregeln bezeichnen in dieser Anmeldung die Abkürzungen für die Aminosäuren, wie z.B. die obengenannten, die freie Säure und, wenn nicht anders angegeben, deren L-Konfiguration. Die $\alpha$-Aminogruppe ist jeweils an der linken Seite der Abkürzung, die Carboxylgruppe an der rechten Seite zu denken. Das Fehlen eines H-Atoms in der $\alpha$-Amino-Gruppe wird durch einen links an der Abkürzung für die Aminosäure stehenden Bindestrich gekennzeichnet, das Fehlen einer HO-Gruppe in der Carboxylgruppe durch einen rechts stehenden Bindestrich ausgedrückt. Substituenten in der Seitenkette von freien Aminosäuren oder Resten davon werden unmittelbar hinter das Aminosäuresymbol in Klammern gesetzt.

Nach dem Prioritätsdatum der vorliegenden Anmeldung wurde gefunden, dass die erfindungsgemässen Peptide im menschlichen Organismus als solche vorkommen. Deshalb betrifft die Erfindung in erster Linie die obengenannten Peptide und deren Salze in einer höheren Konzentration als sie in der Natur oder in gegebenenfalls bekannten Extrakten vorkommen. Insbesondere betrifft die Erfindung die obengenannten Peptide in isolierter oder angereicherter Form, in reiner oder im wesentlichen reiner Form, in einer im wesentlichen anderen Umgebung, d.h. mit anderen Beimengungen, z.B. mit beigemischten pharmazeutischen Trägermaterialien, als sie in der Natur vorkommen, in einer zur pharmazeutischen Verwendung geeigneten Form, in charakterisierter Form und/oder in einer Form ausserhalb eines lebenden oder toten Organismus, eines Organs, einer Zelle, eines Gewebes und einer Körperflüssigkeit. Insbesondere betrifft die Erfindung die synthetisch oder gentechnisch hergestellten Peptide der Formel I und deren Salze.

Im obigen Zusammenhang bedeutet der Begriff "isoliert" abgetrennt von anderen Substanzen, insbesondere von anderen chemischen Verbindungen, mit denen die erfindungsgemässen Verbindungen gegebenenfalls in der Natur zusammen vorkommen können. Der Begriff "gereinigt" bedeutet chemischen und/oder physikalischen Reinigungsmethoden unterzogen. Der Begriff "im wesentlichen rein" bedeutet einen Reinheitsgrad von mehr als 50 %.

Die Erfindung betrifft auch die Salze, insbesondere die pharmazeutisch verwendbaren, nicht-toxischen Salze der erfindungsgemässen Peptide. Die obengenannten Peptide können Säureadditionssalze bilden, z.B. mit anorganischen Säuren, insbesondere Mineralsäuren, z.B. Salzsäure, Schwefelsäure oder Phosphorsäure, oder Salze mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsaure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, sowie Methansulfonsäure, Ethansulfonsäure, 2-Hydroxyethansulfonsäure, Ethan-1,2-disulfonsäure, Benzolsulfonsäure, 4-Methyl-benzolsulfonsäure oder Naphthalin-2-sulfonsäure, oder mit anderen sauren organischen Verbindungen, wie Ascorbinsäure.

Die obengenannten Peptide können auch Metall- oder Ammoniumsalze bilden, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxyethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Aminobenzoesäure-2-diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin.

Die obengenannten Peptide können zudem innere Salze bilden.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Die erfindungsgemässen Verbindungen können in an sich bekannter Weise hergestellt werden, z.B. indem man

a) eine, in einer Verbindung mit der Aminosäuresequenz der Formel I vorhandene Amidbindung durch Umsetzen eines Fragments einer solchen Verbindung, das eine reaktionsfähige freie Carboxylgruppe aufweist, oder eines reaktionsfähigen Carbonsäurederivats davon mit dem komplementären Fragment einer solchen Verbindung, das eine freie Aminogruppe aufweist, oder mit einem reaktionsfähigen Derivat davon, wobei in den genannten Fragmenten freie funktionelle Gruppen mit Ausnahme der beiden an der Reaktion teilnehmenden Gruppen gegebenenfalls in geschützter Form vorliegen, bildet und gegebenenfalls vorhandene Schutzgruppen abspaltet, oder

b) Wirtszellen, die mit einem Expressionsvektor transformiert sind, der eine von einer Expressionskontrollsequenz regulierte, für die Aminosäuresequenz der Formel I kodierende DNA-Sequenz enthält, vermehrt, und eine Verbindung mit der Aminosäuresequenz der Formel I, falls notwendig, aus den Wirtszellen freisetzt und isoliert, und wenn notwendig, in einer erfindungsgemäss erhältlichen Verbindung mit einer Sulfoxidgruppierung diese in die Thiogruppe überführt, und, wenn erwünscht, ein verfahrensgemäss erhältliches Salz in die freie Verbindung überführt und/oder eine verfahrensgemäss erhältliche freie Verbindung in ein Salz überführt.

Verfahren a:

Gemäss diesem Verfahren wird als letzte Reaktionsstufe bei der Synthese des Endprodukts eine Amidbindung an beliebiger Stelle der Aminosäuresequenz der Formel I geknüpft. Beim genannten Fragment, das eine freie Carboxylgruppe aufweist, kann es sich sowohl um eine einzelne Aminosäure oder um ein Di-, Oligo- oder Polypeptid handeln. Dasjenige Fragment, das eine freie Aminogruppe aufweist, stellt ebenfalls eine einzelne Aminosäure, ein Di-, Oligo- oder Polypeptid dar.

Vorzugsweise wird die Reaktion so durchgeführt, dass ein reaktionsfähiges Carbonsäurederivat des einen Fragments mit dem komplementären Fragment, das eine freie Aminogruppe aufweist, umgesetzt wird, wobei die Derivatisierung der Carboxylgruppe des Carbonsäurefragments auch in situ erfolgen kann.

Reaktionsfähige Carbonsäurederivate sind in erster Linie reaktionsfähige aktivierte Ester oder reaktionsfähige Anhydride, ferner reaktionsfähige cyclische Amide; dabei können, wie erwähnt, reaktionsfähige Carbonsäurederivate auch in situ gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Diisopropyl- oder N,N'-Dicyclohexyl-carbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode). Aktivierte Ester sind ferner z.B. geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlorphenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), geeignete Thioester, insbesondere gegebenenfalls, z.B. durch Nitro, substituierte Phenyl-thioester (die man z.B. durch Behandeln der entsprechenden Säure mit gegebenenfalls, z. B. durch Nitro, substituierten Thiophenolen, u.a. mit Hilfe der Anhydrid- oder Carbodiimid-Methode, erhalten kann; Methode der aktivierten Thiolester), oder Amino- oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxy-phthalimid oder 1-Hydroxy-1H-benztriazol, z.B. nach der Anhydrid- oder Carbodiimid-Methode, erhalten kann; Methode der aktivierten N-Hydroxyester).

Reaktionsfähige Säureanhydride können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit einem geeigneten Halogenierungsmittel, wie Thionylchlorid, Phosphorpentachlorid oder Oxalylchlorid, erhalten kann; Säurechloridmethode), Azide (die man z.B. aus einem Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydrochinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Wie erwähnt, können die Carbonsäurederivate auch in situ gebildet werden. So kann man z.B. N,N'-disubstituierte Amidinoester in situ bilden, indem man das Gemisch des komplementären Fragments mit der freien Aminogruppe und des Peptidfragments mit freier Carboxylgruppe in Gegenwart eines geeigneten N,N-disubstituierten Carbodiimids, z.B. N,N'-Diisopropyl- oder N,N-Dicyclohexyl-carbodiimid, zur Reaktion bringt. Ferner kann man Amino-oder Amidoester von Säuren in Gegenwart des zu acylierenden Amins bilden, indem man das Gemisch der entsprechenden Säure- und Amino-Ausgangsstoffe in Gegenwart eines N,N'-disubstituierten Carbodiimids, z.B. N,N'-Dicyclohexyl- oder N,N'-Diisopropyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Alternativ kann Verfahrensvariante a) auch so durchgeführt werden, dass man ein Fragment mit freier Carboxylgruppe mit dem komplementären Fragment umsetzt, in dem die Aminogruppe in reaktionsfähiger Form vorliegt; sie kann z.B. durch Umsetzen mit einem Phosphit, z.B. Diethylchlorphosphit, 1,1-Phenylen-chlorphosphit, Ethyl-dichlor-phosphit, Ethylen-chlor-phosphit oder Tetraethylpyrophosphit, oder mit einem geeigneten Silylierungsmittel, wie einem organischen Halogensilan, z.B. Trimethylchlorsilan, aktiviert werden. Die Aminogruppe kann auch durch Bindung an Halogencarbonyl, z.B. Chlorcarbonyl, oder in Form einer Isocyanatgruppe aktiviert sein.

Funktionelle Gruppen in den genannten Fragmenten, die, sofern sie nicht an der Reaktion teilnehmen sollen, vorteilhafterweise in geschützter Form vorliegen, sind insbesondere Carboxy-, Amino- und Hydroxy-, ferner Carbamoyl- und Guanidinogruppen.

Schutzgruppen, ihre Einführung und Abspaltung sind beispielsweise in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974, sowie in Theodora W. Greene, "Protective Groups in Organic Synthesis, John Wiley & Sons, New York 1981, beschrieben. Charakteristisch für Schutzgruppen ist, dass sie leicht, d.h. ohne unerwünschte Nebenreaktionen, z.B. solvolytisch, reduktiv oder photolytisch, abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitrobenzyloxycarbonyl, oder Diphenylmethoxycarbonyl, oder 2-Halogen-niederalkoxycarbonyl, wie 2,2,2-Trichlorethoxycarbonyl, oder Formyl. Weitere Hydroxyschutzgruppen sind z.B. geeignete verethernde Gruppen, wie Trityl, tert.-Niederalkyl, z.B. tert.-Butyl, 2-oxa- oder 2-thia-aliphatische oder -cycloaliphatische Kohlenwasserstoffreste, in erster Linie 1-Niederalkoxyniederalkyl oder 1-Niederalkylthio-niederalkyl, z.B. Methoxymethyl, 1-Methoxy-ethyl, 1-Ethoxy-ethyl, Methylthiomethyl, 1-Methylthioethyl oder 1-Ethylthioethyl, oder 2-Oxa- oder 2-Thiacycloalkyl mit 5-6 Ringatomen, z.B. 2-Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten von Phenylresten z.B. Halogen, wie Chlor, Niederalkoxy, wie Methoxy, und/oder Nitro in Frage kommen. Weitere Hydroxyschutzgruppen sind ebenfalls organische Silyl- oder Stannylreste, die vorzugsweise Niederalkyl, insbesondere Methyl, und/oder Aryl, z.B. Phenyl, als Substituenten enthalten, in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Carboxylgruppen sind vorzugsweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, α-Aryl-niederalkoxycarbonyl mit einem oder zwei Arylresten, wobei diese gegebenenfalls, z.B. durch Niederalkyl, wie tert.-Niederalkyl, z.B. tert.-Butyl, Niederalkoxy, wie Methoxy, Hydroxy, Halogen, z.B. Chlor, Nitro und/oder Phenyl, substituierte Phenylreste darstellen, wie gegebenenfalls, z.B. wie oben erwähnt, substituiertes Benzyloxycarbonyl, z.B. 4-Methoxybenzyloxycarbonyl oder 4-Nitrobenzyloxycarbonyl, Biphenylylniederalkoxycarbonyl, worin Biphenylyl die α-Stellung substituiert, z.B. 2-(p-Biphenylyl)-2-propyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 2,2-Diaryl-ethoxycarbonyl, worin Aryl gegebenenfalls, z.B. durch Nitro, substituiertes Phenyl, wie 4-Nitrophenyl darstellt, wie 2,2-Di-(4-nitro-phenyl)-ethoxycarbonyl, wobei die beiden Aryl-, z.B. Phenylreste auch verbunden sein können, z.B. 2-(9-Fluorenyl)-ethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen und/oder Nitro, substituierten, aliphatischen, araliphatischen, cycloaliphatischen oder aromatischen Kohlenwasserstoffrest, wie gegebenenfalls entsprechend substituiertes Niederalkyl, Phenylnieder alkyl, Cycloalkyl oder Phenyl, bedeuten, z.B. 2-Triniederalkylsilyl-ethoxycarbonyl, wie 2-Trimethylsilyl-ethoxycarbonyl oder 2-(Methyl-di-(n-butyl)-silyl)-ethoxycarbonyl, oder 2-Triarylsilyl-ethoxycarbonyl, wie 2-Triphenylsilyl-ethoxycarbonyl.

Bevorzugte geschützte Carboxylgruppen sind z.B. tert.-Niederalkoxycarbonyl, wie tert.-Butoxycarbonyl, und gegebenenfalls, z.B. wie oben erwähnt, substituiertes Benzyloxycarbonyl, wie 4-Methoxy- oder 4-Nitrobenzyloxycarbonyl, oder Diphenylmethoxycarbonyl, ferner 2-(Trimethylsilyl)-ethoxycarbonyl.

Eine geschützte Aminogruppe kann z.B. in Form einer leicht spaltbaren Acylamino-, Arylmethylamino-, verätherten Mercaptoamino-, 2-Acyl-niederalk-1-en-yl-amino-, Silyl- oder Stannylaminogruppe oder als Azidogruppe vorliegen.

In einer entsprechenden Acylaminogruppe ist Acyl beispielsweise der entsprechende Rest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, z.B. Chlor, Niederalkoxy z.B. Methoxy, Nitro und/oder Phenyl, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor- oder 2,2,2-Trichloracetyl, gegebenenfalls, z.B. durch Halogen, Niederalkoxy, Nitro und/oder Phenyl, substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, α-Arylniederalkoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, Nitro und/oder Phenyl, substituiertes Phenyl darstellen, wie gegebenenfalls substituiertes Benzyloxycarbonyl, z.B. 4-Nitrobenzyloxycarbonyl, Biphenylylniederalkoxycarbonyl, worin Biphenylyl die α-Stellung substituiert, z.B. 2-(p-Biphenylyl)-2-propyloxycarbonyl, oder substituiertes Diphenylmethoxycarbonyl, z.B. Benzhydryloxycarbonyl oder Di-(4-methoxyphenyl)-methoxycarbonyl, 2,2-Diaryl-ethoxycarbonyl, worin Aryl gegebenenfalls, z.B. durch Nitro, substituiertes Phenyl, wie 4-Nitrophenyl darstellt, wie 2,2-Di-(4-nitro-phenyl)-ethoxycarbonyl, wobei die beiden Aryl-, z.B. Phenylreste auch verbunden sein können, z.B. 2-(9-Fluorenyl)-ethoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B.

Dicyclohexylphosphoryl, gegebenenfalls substituiertes Diphenylphosphoryl, z.B. Diphenylphosphoryl, gegebenenfalls, z.B. durch Nitro, substituiertes Di-(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellen kann, sind die Arylreste insbesondere gegebenenfalls substituierte Phenylreste. Solche Gruppen sind beispielsweise Benzyl-, Diphenylmethyl- und besonders Tritylamino.

Eine veretherte Mercaptogruppe in einer durch einen solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro, substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B 1-Ethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor-oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, 2-(9-Fluorenyl)-ethoxycarbonyl, oder 2-Halogenniederalkoxycarbonyl, wie 2,2,2-Trichlorethoxycarbonyl, ferner Trityl oder Formyl.

Unsubstituierte Carbamoylgruppen sind z.B. in Form von N-(9-Xanthenyl)-oder in Form von N-(Mono-, Di- oder Triarylmethyl)-Derivaten geschützt, wobei Aryl insbesondere unsubstituiertes oder bis zu 5 gleiche oder verschiedene Substituenten, vorzugsweise Niederalkyl, wie Methyl, oder Niederalkoxy, wie Methoxy, enthaltendes Phenyl bedeutet. Als Beispiele für solche Arylmethyl-Schutzgruppen seien genannt: 4-Methoxy-benzyl, 2,4,6-Trimethoxy-benzyl, Diphenylmethyl, Di-(4-methoxy-phenyl)-methyl, Di-(4-methyl-phenyl)-methyl und (4-methyl-phenyl)-([polymerer Träger]-phenyl)-methyl. Eine bevorzugte Carbamoylschutzgruppe ist Trityl.

Guanidinogruppen können z.B. mittels geeignet substituierter Sulfonylgruppen, wie Arylsulfonyl, worin Aryl gegebenenfalls substituiertes, z.B. Niederalkyl, wie Methyl, enthaltendes Phenyl, oder gegebenenfalls, z.B. durch Niederalkyl, wie Methyl, substituiertes, über ein aromatisches Kohlenstoffatom gebundenes Benzoheterocyclyl, wie Chromanyl, darstellt, wie 4-Methoxyphenylsulfonyl oder 2,2,5,7,8-Pentamethyl-6-chromanylsulfonyl geschützt sein.

Als Schutzgruppe, insbesondere als Carboxylschutzgruppe, im Sinne der vorliegenden Anmeldung ist auch ein mit der zu schützenden funktionellen Gruppe, insbesondere einer Carboxylgruppe, verbundener polymerer Träger zu verstehen, der sich in erster Linie für die sog. Merrifield-Peptidsynthese eignet und leicht abspaltbar ist. Ein solcher polymerer Träger ist z.B. vorzugsweise ein durch Copolymerisation mit Divinylbenzol schwach vernetztes Polystyrolharz, das zur reversiblen Bindung von Aminosäure- bzw. Peptidresten geeignete Brückenglieder trägt. Diese sind, insbesondere im Zusammenhang mit dem obgenannten leicht vernetzten Polystyrolharz in erster Linie direkt mit aromatischen Resten des Polystyrolharzes verbundene und gegebenenfalls substituierte Methylengruppen. Substituenten von letzteren sind vorzugsweise über Ether- oder Estergruppierungen mit den Methylengruppen verbunden und enthalten geeignete funktionelle Gruppierungen, die zusammen mit funktionellen Gruppen, insbesondere Carboxylgruppen, des Aminosäure- oder Peptidfragmentes geschützte Gruppen, insbesondere entsprechende Carboxylgruppen, wie veresterte Carboxylgruppen, bilden können. Solche Brückenglieder sind z.B. die zweiwertigen Reste von gegebenenfalls in α-Stellung gegebenenfalls, z.B. durch Niederalkoxy, wie Methoxy, z.B. in o-und/oder p-Stellung subsituiertes Phenyl, enthaltenden 4-Methoxy-benzylalkoholen, wobei das Kohlenstoffatom der 4-Methoxygruppe mit einem Phenylrest des Polystyrolharzes direkt verbunden ist, und die benzylische Hydroxygruppe die Carboxylfunktion der Aminosäure oder des Peptidfragments verestert.

Die Reaktion zur Bildung der Amidbindung kann in an sich bekannter Weise durchgeführt werden, wobei die Reaktionsbedingungen in erster Linie davon abhängen, ob und wie die an der Reaktion teilnehmende Carboxylgruppe aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe in Form eines Anhydrids vorliegt, auch ein geeignetes Säure-bindendes Mittel sein kann, unter Kühlen oder Erwärmen, z.B. in einem Temperaturbereich von etwa -30°C bis etwa +150°C, insbesondere von +10°C bis +70°C, vorzugsweise von Raumtemperatur (etwa +20°C) bis +50°C, in einem geschlossenen Reaktionsgefäss und/oder in der Atmosphäre eines Inertgases, z.B. Stickstoff.

Uebliche Kondensationsmittel sind z.B. Carbodiimide, beispielsweise N,N′-Diethyl-, N,N′-Diisopropyl-, N,N′-Dicyclohexyl- oder N-Ethyl-N′-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3′-sulfonat und 2-tert.-Butyl-5-methylisoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Die oben erwähnte Merrifield-Peptidsynthese eignet sich insbesondere für eine halb- oder vollautomatische Synthese von Verbindungen mit der Aminosäuresequenz der Formel I, in welcher Aminosäuren und/oder Peptidfragmente, worin die an der Reaktion nicht teilnehmenden funktionellen Gruppen üblicherweise in geschützter Form vorliegen, über Amidgruppierungen ohne Isolierung der gebildeten Peptidfragmente miteinander verknüpft werden. Dabei ist eine der funktionellen Gruppen, normalerweise die im Endpeptid vorhandene terminale Carboxylgruppe, gegebenenfalls über ein Brückenglied, wie beschrieben, mit einem geeigneten polymeren Träger verbunden. Im Prinzip wird diese Verfahrensvariante analog wie die übliche Synthese von Peptiden durchgeführt, wobei darauf geachtet wird, dass die Freisetzung der an der Reaktion teilzunehmenden funktionellen Gruppe im schon synthetisierten Peptidfragment, das den polymeren Trägerteil enthält, üblicherweise die terminale Aminogruppe, aus der geschützten Gruppe jeweils unter Bedingungen erfolgt, unter denen die Schutzgruppen der an der Reaktion nicht teilnehmenden funktionellen Gruppen erhalten bleiben.

Die Abspaltung der Carboxyl-, Amino-, Hydroxy-, Carbonsäureamid-, Carbamoyl- und/oder Guanidinoschutzgruppen erfolgt in an sich bekannter Weise, z.B. mittels Solvolyse, insbesondere Hydrolyse (unter sauren oder basischen Bedingungen), Alkoholyse, Acidolyse oder Basenbehandlung, oder mittels Reduktion, insbesondere Hydrogenolyse oder chemische Reduktion, gegebenenfalls stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl acidolytisch, z.B. durch Behandeln mit einer geeigneten Säure, wie einer gegebenenfalls Halogen enthaltenden Niederalkancarbonsäure, z.B. Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. 2,2-Diaryl-ethoxycarbonyl- oder 2-(9-Fluorenyl)-ethoxycarbonylgruppen lassen sich unter milden basischen Bedingungen, z.B. durch Behandlen mit Piperidin, spalten. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silylethoxycarbonyl kann auch durch Behandeln mit einem, das Fluoridanion liefernden Salz der Fluorwaserstoffsäure, wie einem Alkalimetallfluorid, z.B. Natrium-oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung einer 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, wie einer gegebenenfalls, z.B. durch Halogen, wie Fluor, subsitiuierten Niederalkancarbonsäure, z.B. Ameisensäure oder Trilfluoressigsäure, und 2,2-Diaryl-ethoxy carbonylamino, wie 2,2-Di-(4-Nitrophenyl)-ethoxycarbonylamino, ferner 2-(9-Fluorenyl)-ethoxycarbonylamino durch Behandeln mit einer geeigneten Base, wie einem aliphatischen, vorzugsweise sekundären Amin, z.B. Piperidin, gespalten werden. Die Aminogruppe kann aus gegebenenfalls substituiertem Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, aus gegebenenfalls substituiertem Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und aus einer organischen Silylaminogruppe z.B. mittels Hydrolyse oder Alkoholyse freigesetzt werden. Eine durch 2-Halogenacetyl, z.B. 2-Chloracetyl, geschützte Aminogruppe kann z.B. durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwaserstoffsäure, wie oben im Zusammenhang mit der Freisetzung einer entsprechend geschützten Carboxylgruppe beschrieben, in die freie Aminogruppe übergeführt werden.

In Form einer Azidogruppe vorliegendes Amino kann z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung kann vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt werden.

Eine durch eine geeignete Acylgruppe, eine organische Silylgruppe oder durch gegebenenfalls substituiertes 1-Phenylniederalkyl geschützte Hydroxygruppe kann analog einer entsprechend geschützten Aminogruppe freigesetzt werden. Eine durch 2,2-Dichloracetyl geschützte Hydroxygruppe kann z.B. durch basische Hydrolyse, eine durch tert.-Niederalkyl, z.B. tert.-Butyl, oder durch einen 2-oxa- oder 2-thia-aliphatischen oder -cycloaliphatischen Kohlenwasserstoffrest veretherte Hydroxygruppe durch Acidolyse, z.B. durch Behandeln mit einer Mineralsäure oder einer starken Carbonsäure, z.B. Trifluoressigsäure, freigesetzt werden.

Eine durch 9-Xanthenyl geschützte Carbonsäureamidgruppe kann z.B. durch Behandeln mit Bromwasserstoff in Eisessig oder mit Fluorwasserstoff in Gegenwart von Anisol freigesetzt werden. Eine durch Mono-, Di- oder Triarylmethyl geschützte Carbonsäureamidgruppe kann z.B. durch Behandeln mit Fluorwasserstoff in Gegenwart von Anisol freigesetzt werden; ferner kann z.B. eine Diphenylmethyl-Schutzgruppe hydrogenolytisch in Gegenwart eines Palladium-auf-Kohle-Katalysators und eine Di-(4-methoxy-phenyl)-methyl-Schutzgruppe, sowie eine 2,4,6-Trimethoxy-benzyl-Schutzgruppe z.B. durch Behandeln mit Trifluoressigsäure abgespalten werden.

Mittels organischen Sulfonylgruppen geschützte Guanidinogruppen, wie 4-Methylphenylsulfonyl- oder 2,2,5,7,8-Pentamethyl-6-chromanylsulfonylguanidino können z.B. durch Behandeln mit einer geeigneten Säure, wie Trifluoressigsäure, freigesetzt werden.

Die Spaltung einer geschützten funktionellen Gruppe, insbesondere einer entsprechenden Carboxylgruppe, deren Schutzgruppe gleichzeitig als Trägermaterial in der erwähnten Merrifield-Peptidsynthese dient, kann in an sich bekannter Weise, z.B. wie oben beschrieben, erfolgen. Eine entsprechend veresterte Carboxylgruppe, die über ein geeignetes Brückenglied mit dem polymeren Trägermaterial verbunden ist, wird je nach Art des Brückengliedes gespalten. So kann z.B. eine über eine Estergruppierung mit einem aktivierten benzylisches Brückenglied an das polymere Trägermaterial gebundene Carboxylgruppe, z.B. eine 4-Methoxybenzyloxycarbonylgruppe, worin das Kohlenstoffatom der Methoxygruppe z.B. mit einem Phenylrest des leicht mit Divinylbenzol vernetzten Polystyrolharzes verbunden ist, analog den oben erwähnten gegebenenfalls substituierten Benzyloxycarbonylgruppen, z.B. durch Behandeln mit einer geeigneten Säure, wie Trifluoressigsäure, freigesetzt werden.

Wenn erwünscht, können beim Vorhandensein von mehreren geschützten funktionellen Gruppen die Schutzgruppen so gewählt werden, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator.

Verfahren b)
Die zur Herstellung der erfindungsgemässen Polypeptide verwendeten transformierten Wirtszellen werden hergestellt, indem man
- eine für ein erfindungsgemässes Polypeptid kodierende DNA herstellt,
- einen diese DNA enthaltenden Hybridvektor herstellt, und
- einen geeigneten Wirtsstamm mit besagtem Hybridvektor transformiert.

Bei der für ein erfindungsgemässes Polypeptid kodierenden DNA handelt es sich um die entsprechende, aus einer humanen Genbank mit Hilfe bekannter Verfahren isolierte chromosomale DNA, um die über den mRNA-Weg isolierte cDNA oder um die entsprechende synthetische DNA. Letztere wird nach allgemein bekannten Verfahren, z.B. nach dem von S.A. Narang [Tetrahedron $\underline{39}$, 3 (1983)] oder nach dem in der Europäischen Patentanmeldung Nr. 146785 beschriebenem Verfahren, insbesondere unter Verwendung kommerziell erhältlicher DNA-Synthesemaschinen, hergestellt.

Eine entsprechende für PCATFP kodierende DNA hat die Sequenz
$Z_1-Y_{13}-Y_1-Y_{15}-Y_{14}-Y_2-Y_{16}-Y_1-Y_{11}-Y_7-Y_{16}-Y_{16}-Y_{15}-Y_1-Y_4-Y_{15}-Y_1-Y_9-Y_{11}-Y_{16}-Y_7-Y_4-Y_7-Y_1-Y_2-Y_{11}-Y_{11}-Y_{11}-Y-$
$1-Y_1-Y_{11}-Y_3-Y_6-Y_4-Y_5-Y_3-Y_6-Y_{13}-Y_{12}-Y_1-Y_{16}-Y_7-Y_{11}-Y_7-Y_6-Y_7-Y_6-Y_7-Y_2-Y_7-Y_8-Y_{16}-Y_{16}-Y_{11}-Y_4-Y_{16}-Y_{15}-Y_2-$

$Y_{16}$-$Y_{17}$-$Z_2$

während eine für PCGRPATFP kodierende DNA die Sequenz

$Z_1$-$Y_{13}$-$Y_1$-$Y_{15}$-$Y_{14}$-$Y_2$-$Y_{16}$-$Y_1$-$Y_{11}$-$Y_7$-$Y_{16}$-$Y_{16}$-$Y_{15}$-$Y_1$-$Y_4$-$Y_{15}$-$Y_1$-$Y_9$-$Y_{11}$-$Y_{16}$-$Y_7$-$Y_4$-$Y_7$-$Y_1$-$Y_2$-$Y_{11}$-$Y_{11}$-$Y_{11}$-$Y_{1}$-$Y_1$-$Y_{11}$-$Y_3$-$Y_6$-$Y_4$-$Y_5$-$Y_3$-$Y_6$-$Y_{13}$-$Y_{12}$-$Y_1$-$Y_{16}$-$Y_7$-$Y_{11}$-$Y_7$-$Y_6$-$Y_7$-$Y_6$-$Y_7$-$Y_2$-$Y_7$-$Y_8$-$Y_{16}$-$Y_2$-$Y_{10}$-$Y_{10}$-$Y_1$-$Y_6$-$Y_{17}$-$Z_2$,

aufweist, worin

$Y_1$ für Alanin (Ala) kodiert und GCT, GCC, GCA oder GCG ist,

$Y_2$ für Arginin (Arg) kodiert und CGT, CGC, CGA, CGG, AGA oder AGG ist,

$Y_3$ für Valin (Val) kodiert und GTT, GTC, GTA oder GTG ist,

$Y_4$ für Asparaginsäure (Asp) kodiert und GAT oder GAC ist,

$Y_5$ für Tyrosin(Tyr) kodiert und TAT oder TAC ist,

$Y_6$ für Glutamin (Gln) kodiert und CAA oder CAG ist,

$Y_7$ für Glutaminsäure (Glu) kodiert und GAA oder GAG ist,

$Y_8$ für Glycin (Gly) kodiert und GGT, GGC, GGA oder GGG ist,

$Y_9$ für Threonin (Thr) kodiert und ACT, ACC, ACA oder ACG ist,

$Y_{10}$ für Isoleucin (Ile) kodiert und ATT, ATC oder ATA ist,

$Y_{11}$ für Leucin (Leu) kodiert und TTA, TTG, CTT, CTC, CTA oder CTG ist,

$Y_{12}$ für Lysin (Lys) kodiert und AAA oder AAG ist,

$Y_{13}$ für Methionin (Met) kodiert und ATG ist,

$Y_{14}$ für Phenylalanin (Phe) kodiert und TTT oder TTC ist,

$Y_{15}$ für Prolin (Pro) kodiert und CCT, CCC, CCA oder CCG ist,

$Y_{16}$ für Serin (Ser) kodiert und TCT, TCC, TCA, TCG, AGT oder AGC ist,

$Y_{17}$ für TAA, TAG oder TGA (Stopcodon) steht, und

$Z_1$ und $Z_2$ flankierende DNA-Reste, enthaltend 5 bis 100, insbesondere 5 bis 15 Nukleotide darstellen, die jeweils eine Restriktionsenzym-Erkennungssequenz enthalten.

In erster Linie betrifft die Erfindung für PCATFP und PCGRPATFP kodierende DNAs, welche von E. coli oder S. cerevisiae bevorzugte Triplets enthalten.

Eine bevorzugte, für PCATFP kodierende DNA hat die Sequenz (mit angedeuteten Retriktionsenzym-Schnittstellen)

```
5'      MetAlaProPheArgSerAlaLeuGluSerSerProAlaAspProAlaThr
  CTGGAATTCATGGCTCCGTTCCGTTCTGCTCTGGAATCTTCTCCGGCTGACCCGGCTACC
  GACCTTAAGTACCGAGGCAAGGCAAGACGAGACCTTAGAAGAGGCCGACTGGGCCGATGG
3' ------

   EcoRI


  LeuSerGluAspGluAlaArgLeuLeuLeuAlaAlaLeuValGlnAspTyrValGlnMet
  CTGTCTGAAGACGAAGCTCGTCTGCTGCTAGCTGCTCTGGTTCAGGACTACGTTCAGATG
  GACAGACTTCTGCTTCGAGCAGACGACGATCGACGAGACCAAGTCCTGATGCAAGTCTAC
                      -------

                  NheI


  LysAlaSerGluLeuGluGlnGluGlnGluArgGluGlySerSerLeuAspSerProArg
  AAAGCTTCTGAACTGGAACAGGAACAGGAACGTGAAGGTTCTTCTCTGGACTCTCCGCGT
  TTTCGAAGACTTGACCTTGTCCTTGTCCTTGCACTTCCAAGAAGAGACCTGAGAGGCGCA


  SerNON      3'
  TCTTAGGATCCTG
  AGAATCCTAGGAC
        ------5'              (III)

   BamHI
```

Das Oligonucleotid der Formel III wird beispielsweise nach dem in der Europäischen Patentanmeldung Nr. 168342 beschriebenen Verfahren hergestellt, indem man die in Formel III unterstrichenen Partialsequenzen chemisch synthetisiert, kinasiert, mit DNA-Polymerase und den vier Nucleosidtriphosphaten zu den Dupleces auffüllt und über die eingebauten Restriktionsschnittstellen zum PCATFP-Gen ligiert.

Die Erfindung betrifft weiterhin Expressionsvektoren, welche eine von einer Expressionskontrollsequenz regulierte für PCATFP oder PCGRPATFP kodierende DNA-Sequenz enthalten.

Die Expressionsvektoren der vorliegenen Erfindung werden z.B. hergestellt, indem man in eine Vektor-DNA, welche eine Expressionskontrollsequenz enthält, eine für ein erfindungsgemässes Polypeptid kodierende DNA-Sequenz derart einfügt, dass die Expressionskontrollsequenz besagte DNA-Sequenz reguliert.

Die Wahl eines geeigneten Vektors ergibt sich aus der zur Transformation vorgesehenen Wirtszelle. Geeignete Wirte sind beispielsweise Mikroorganismen, wie Hefen, z.B. Saccharomyces cerevisiae, und Bakterienstämme, vor allem Stämme von Escherichia coli, Bacillus subtilis, Bacillus stearothermophilus, Pseudomonas, Haemophilus, Streptococcus und andere, ferner Zellen höherer Organismen, insbesondere etablierte humane oder tierische Zellinien.

Grundsätzlich sind alle Vektoren geeignet, welche die heterologen, für die erfindungsgemässen Polypeptide kodierenden DNA-Sequenzen in dem gewählten Wirt zu replizieren und exprimieren vermögen.

Beispiele für Vektoren, die zur Expression eines Gens in einem E. coli-Stamm geeignet sind, sind Bacteriophagen, z.B. Derivate des Bacteriophagen λ, oder Plasmide, wie insbesondere das Plasmid colE1 und seine Derivate, z.B. pBR322. Geeignete Vektoren enthalten ein intaktes Replicon und ein Markierungsgen, welches die Selektion und Identifizierung der mit den Expressionsplasmiden transformierten Mikroorganismen auf Grund eines phänotypischen Merkmals ermöglicht. Geeignete Markierungsgene verleihen dem Mikrooganismus beispielsweise Resistenz gegenüber Schwermetallen, Antibiotika und dergleichen. Weiterhin enthalten bevorzugte Vektoren ausserhalb der Replicon- und Markierungsgen-Regionen Erkennungssequenzen für Restriktionsendonucleasen, so dass an diesen Stellen die für das erfindungsgemässe Polypeptid kodierende DNA-Sequenz und gegebenenfalls die Expressions-Kontrollsequenz eingefügt werden können. Das Plasmid pBR322 enthält ein intaktes Replicon und gegen Tetracyclin und Ampicillin Resistenz verleihende Markierungsgene (tet$^R$ und amp$^R$).

Mehrere Expressionskontrollsequenzen können zur Regulation der Polypeptid-Expression eingesetzt werden. Insbesondere werden Expressionskontrollsequenzen stark exprimierter Gene der zu transformierenden Wirtszelle verwendet. Im Falle von pBR322 als Hybridvektor und E. coli als Wirtsmikroorganismus sind beispielsweise die Expressionskontrollsequenzen (welche unter anderem den Promoter und die ribosomale Bindungsstelle enthalten) des Lactoseoperons, Tryptophanoperons, Arabinoseoperons und dergleichen, des β-Lactamasegens, die entsprechenden Sequenzen des Phage λN-Gens oder des Phage fd-Schichtproteingens und andere geeignet. Während der Promoter des β-Lactamasegens (β-lac-Gen) bereits im Plasmid pBR322 enthalten ist, müssen die übrigen Expressionskontrollsequenzen in das Plasmid eingeführt werden.

Zur Replikation und Expression in Hefe geeignete Vektoren enthalten einen Hefe-Replikationsstart und einen selektiven genetischen Marker für Hefe. Hybridvektoren, die einen Hefe-Replikationsstart enthalten, z.B. das chromosomale autonom replizierende Segment (ars), bleiben nach der Transformation innerhalb der Hefezelle extrachromosomal erhalten und werden bei der Mitose autonom repliziert. Ferner können Hybridvektoren, die der Hefe-2μ-Plasmid-DNA homologe Sequenzen enthalten, verwendet werden. Solche Hybridvektoren werden durch Rekombination innerhalb der Zelle bereits vorhandenen 2μ-Plasmiden einverleibt oder replizieren autonom. 2μ-Sequenzen sind besonders für Plasmide mit grosser Transformationshäufigkeit geeignet und gestatten eine hohe Kopienzahl. Geeignete Markierungsgene für Hefe sind vor allem solche, die dem Wirt antibiotische Resistenz verleihen oder, im Fall von auxotrophen Hefemutanten, Gene, die Wirtsdefekte komplementieren. Entsprechende Gene. verleihen z.B. Resistenz gegen das Antibiotikum Cycloheximid oder sorgen für Prototrophie in einer auxotrophen Hefemutante, z.B. das URA3-, LEU2-, HIS3- oder vor allem das TRP1-Gen. Vorzugsweise enthalten Hefe-Hybridvektoren weiterhin einen Replikationsstart und ein Markierungsgen für einen bakteriellen Wirt, insbesondere E. coli, damit die Konstruktion und die Klonierung der Hybridvektoren und ihrer Vorstufen in einem bakteriellen Wirt erfolgen kann. Für die Expression in Hefe geeignete Expressionskontrollsequenzen sind beispielsweise diejenigen des TRP1-, ADHI- oder PHO5-Gens, ferner im glykolytischen Abbau involvierte Promoter, z.B. der PGK- und der GAPDH-Promoter.

Insbesondere betrifft die Erfindung zur Replikation und phänotypischen Selektion befähigte Expressionsvektoren, welche eine Expressionskontrollsequenz und eine für eine der erfindungsgemässen Polypeptide kodierende DNA-Sequenz enthalten, wobei besagte DNA-Sequenz mitsamt Transkriptionsstartsignal und -terminationssignal sowie Translationsstartsignal und -stopsignal in besagtem Expressionsplasmid unter Regulation besagter Expressionskontrollsequenz so angeordnet ist, dass in einer mit besagtem Expressionsplasmid transformierten Wirtszelle die erfindungsgemässen Polypeptide exprimiert werden.

Um eine effektive Expression zu erreichen, muss das Gen richtig (in "Phase") mit der Expressionskontrollsequenz angeordnet sein. Es ist vorteilhaft, die Expressionskontrollsequenz in den Bereich zwischen dem Haupt-mRNA-Start und dem ATG der Genkodiersequenz, welche natürlich mit der Expressionskontrollsequenz verknüpft ist (z.B. die β-lac-Kodiersequenz bei Verwendung des β-lac-Promoters), mit dem Gen, welches vorzugsweise sein eigenes Translationsstartsignal (ATG) und Translationsstopsignal (z.B. TAG) mitbringt, zu verknüpfen. Dadurch wird eine effektive Transkription und Translation gewährleistet.

Beispielsweise wird ein Vektor, insbesondere pBR322, mit einer Restriktionsendonuclease geschnitten und, gegebenenfalls nach Modifikation des so gebildeten linearisierten-Vektors, eine mit entsprechenden Restriktionsenden versehene Expressionskontrollsequenz eingeführt. Die Expressionskontrollsequenz enthält am 3'-Ende (in Translationsrichtung) die Erkennungssequenz einer Restriktionsendonuclease, sodass der die Expressionskontrollsequenz bereits enthaltende Vektor mit besagtem Restriktionsenzym verdaut und das mit passenden Enden versehene, für eine der erfindungsgemässen Polypeptide kodierende Gen eingesetzt werden kann. Dabei entsteht ein Gemisch von zwei Hybridplasmiden, welche das Gen in richtiger bzw. in falscher Orientierung enthalten. Vorteilhaft ist es, den die Expressionskontrollsequenz bereits enthaltenden Vektor noch mit einer zweiten Restriktionsendonuclease innerhalb der Vektor-DNA zu spalten und in das entstandene Vektor-Fragment das mit richtigen Enden versehene, für eine der erfindungsgemässen Polypeptide kodierende Gen einzusetzen. Alle Operationen am Vektor erfolgen vorzugsweise in einer Weise, dass die Funktion des Replicons und zumindest eines Markierungsgens nicht beeinträchtigt wird.

Die Vektorkonstruktion kann auch eine Signalsequenz enthalten, welche operabel an die Expressionskontrollsequenz geknüpft ist und bezüglich dem für das erfindungsgemässe Polypeptid kodierenden Gen das richtige Leseraster ("reading frame") aufweist. Geeignete Signalsequenzen sind beispielsweise die OmpA-, Ipp- and β-lac-Signalsequenzen bei Verwendung von E. coli als Wirtsmikroorganismus und die Invertase-, α-Faktor- und PH05-Signalsequenzen bei Verwendung von S. cerevisiae als Wirtsmikroorganismus.

Die Transformation der Wirtszellen mit den erfindungsgemässen Expressionsplasmiden erfolgt nach bekannten Verfahren. Die Isolierung der transformierten Wirtszellen erfolgt vorteilhaft aus einem selektiven Nährmedium, dem das Biocid zugesetzt wird, gegen welches das im Expressionsplasmid enthaltene Markierungs-Gen Resistenz verleiht. Wenn, wie bevorzugt, die Expressionsplasmide das amp$^R$-Gen enthalten, wird dem Nährmedium demgemäss Ampicillin zugesetzt. Zellen, welche das Expressionsplasmid nicht enthalten, werden in einem solchen Medium abgetötet.

Die Erfindung betrifft ebenfalls die auf dem genannten Weg erhältlichen transformierten Wirtszellen.

Die Kultivierung der erfindungsgemässen transformierten Wirtszellen gemäss Verfahren b) erfolgt in an sich bekannter Weise. So können für die Kultivierung der erfindungsgemässen, transformierten Wirtsmikroorganismen verschiedene Kohlenstoffquellen verwendet werden. Beispiele bevorzugter Kohlenstoffquellen sind assimilierbare Kohlenhydrate, wie Glucose, Maltose, Mannit oder Lactose, oder ein Acetat, das entweder allein oder in geeigneten Gemischen verwendet werden kann. Geeignete Stickstoffquellen sind beispielsweise Aminosäuren, wie Casaminosäuren, Peptide und Proteine und ihre Abbauprodukte, wie Trypton, Pepton oder Fleischextrakte; weiterhin Hefeextrakte, Malzextrakt, wie auch Ammoniumsalze, z.B. Ammoniumchlorid, -sulfat oder -nitrat, die entweder allein oder in geeigneten Mischungen verwendet werden können. Anorganische Salze, die ebenfalls verwendet werden können, sind z.B. Sulfate, Chloride, Phosphate und Carbonate von Natrium, Kalium, Magnesium und Calcium.

Weiterhin enthält das Medium z.B. wachstumsfördernde Substanzen, wie Spurenelemente, z.B. Eisen, Zink, Mangan und dergleichen, und vorzugsweise Substanzen, die einen Selektionsdruck ausüben und das Wachstum von Zellen, die das Expressionsplasmid verloren haben, verhindern. So wird dem Medium beispielsweise Ampicillin zugesetzt, wenn das Expressionsplasmid ein amp$^R$-Gen enthält. Ein solcher Zusatz von antibiotisch wirksamen Substanzen bewirkt auch, dass kontaminierende, Antibiotikaempfindliche Mikroorganismen abgetötet werden.

Die Kultivierung erfolgt nach an sich bekannten Verfahren. Die Kultivierungsbedingungen, wie Temperatur, pH-Wert des Mediums und Fermentationszeit, werden so ausgewählt, dass maximale Polypeptid-Titer erhalten werden. So wird ein E. coli- oder ein Hefe-Stamm bevorzugt unter aeroben Bedingungen in submerser Kultur unter Schütteln oder Rühren bei einer Temperatur von etwas 20 bis 40°C, vorzugsweise etwa 30°C, und einem pH-Wert von 4 bis 8, vorzugsweise bei pH 7, während etwa 4 bis 20 h, vorzugsweise 8 bis 12 h, kultiviert.

Wenn die Zelldichte einen ausreichenden Wert erreicht hat, wird die Kultivierung abgebrochen und das erfindungsgemässe Polypeptid aus den Zellen des Mikroorganismus freigesetzt. Zu diesem Zweck werden die Zellen zerstört, z.B. durch Behandlung mit einem Detergens, wie SDS oder Triton, oder mit Lysozym oder einem gleichartig wirkenden Enzym lysiert. Alternativ oder zusätzlich kann man mechanische Kräfte, wie Scherkräfte (z.B. X-Presse, French-Presse, Dyno-Mill) oder Schütteln mit Glasperlen oder Aluminiumoxid, oder abwechselndes Einfrieren, z.B. in flüssigem Stickstoff, und Auftauen, z.B. auf 30° bis 40°C, sowie Ultraschall zum Brechen der Zellen anwenden. Die resultierende Mischung, die Proteine, Nucleinsäuren und andere Zellbestandteile enthält, wird nach der Zentrifugation in an sich bekannter Weise an Proteinen angereichert. So wird z.B. der grösste Teil der Nicht-Protein-Bestandteile durch Polyäthylenimin-Behandlung abgetrennt und die Proteine einschliesslich der erfindungsgemässen Polypeptide z.B. durch Sättigen der Lösung mit Ammoniumsulfat oder mit anderen Salzen ausgefällt. Bakterielle Proteine können auch mittels Ansäuern mit Essigsäure (z.B. 0,1 %, pH 4-5) ausgefällt werden. Weitere Reinigungsschritte umfassen bespielsweise chromatographische Verfahren, wie Ionenaustauschchromatographie, Gelpermeationschromatographie, Partitionschromatographie, HPLC, Umkehrphasen-HPLC und dergleichen. So erfolgt die Auftrennung der Mischbestandteile durch Dialyse, nach Ladung mittels Gel- oder trägerfreier Elektrophorese, nach Molekülgrösse mittels einer geeigneten Sephadex-Säule, durch Affinitätschromatographie, z.B. mit Antikörpern, insbesondere monoklonalen Antikörpern, oder mit Thrombin gekuppelt an einen geeigneten Träger zur Affinitätschromatographie, oder durch weitere bekannte Verfahren.

Beispielsweise kann man zur Isolierung eines exprimierten erfindungsgemässen Polypeptids wie folgt vorgehen:

Abtrennung der Zellen aus der Kulturlösung mittels Zentrifugation; Herstellung eines Rohextrakts durch Zerstören der Zellen, z.B. durch Behandlung mit einem lysierenden Enzym und/oder abwechselndem Einfrieren und Wiederauftauen; Abzentrifugieren der unlöslichen Bestandteile; Ausfällen der DNA durch Zugabe von Polyethylenimin; Fällen der Proteine durch Ammoniumsulfat; Ionenautauschchromatographie

11

und/oder Umkehrungsphasen-HPLC; Entsalzen der so gewonnen Lösung mittels Dialyse oder Chromatographie an einem geeigneten Ionenaustauscher, z.B. Sephadex G25 oder Sephadex G10.

Zum Nachweis der erfindungsgemässen Polypeptide kann z.B. der Test mit geeigneten Antikörpern, z.B. aus Hybridomazellen erhältliche monoklonalen Antikörpern, herangezogen werden.

Ueblicherweise erhält man nach Verfahren b) die Peptide der Formel I, worin R für Wasserstoff steht, insbesondere bei Exprimierung in Hefe. In bestimmten Wirtszellen, z.B. E. coli, kann jedoch eine N-terminale Acetylierung erfolgen, so dass man Peptide der Formel I, worin R für Acetyl steht, erhält.

In erfindungsgemäss erhältlichen Peptiden der Formel I mit einem, eine Sulfoxidgruppe enthaltenden Methioninrest wird eine solche in an sich bekannter Weise mittels Reduktion in die Thiogruppe übergeführt. Dabei verwendet man geeignete Reduktionsmittel, vorzugsweise Iodidsalze, wie Ammoniumiodid, üblicherweise in Gegenwart eines Verdünnungsmittel, wobei entstandenes Iod, z.B. durch Behandeln mit Ascorbinsäure, entfernt werden kann.

Salze von Verbindungen der Formel I können in an sich bekannter Weise hergestellt werden. So kann man Salze von Verbindungen der Formel I, die mehr saure als basische Gruppen aufweisen, z.B. durch Behandeln mit Metallverbindungen, wie Alkalimetallsalzen von geeigneten organischen Carbonsäuren, z.B. dem Natriumsalz der α-Ethyl-capronsäure, oder anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche je eine freie saure und eine basische Gruppe enthalten, können z.B. durch Neutrali sieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditonssalze z.B. durch Behandeln mit einem geeigneten basischen Mittel.

Die Peptide der Formel I weisen wertvolle pharmakologische Eigenschaften auf und können entsprechend verwendet werden. Behandelt man z.B. in vitro Calvarienzellen von Hühnerembryonen oder frischgeborenen Ratten mit Peptiden der Formel I, so kann eine Stimulierung der Zellvermehrung anhand des Anstiegs des Einbaus von Tritium-markiertem Thymidin in die DNA festgestellt werden. Diese Stimulierung kann mit Konzentrationen von etwa 1 bis etwa 1000 nmol/l, am ausgeprägtesten mit Konzentrationen von etwa 100 nmol/l erzielt werden, wobei der Effekt in An- oder in Abwesenheit von Serum im Kulturmedium festgestellt werden kann. Die stimulierende Wirkung der Peptide der Formel I ist spezifisch für Osteoblasten; so reagieren z.B. Hautfibroblasten von Ratten nicht. Im Gegensatz zu Calcitonin bzw. Calcitoningen-verwandtem Peptid, verhindern die Peptide der Formel I die Knochenresorption nicht, was in vitro am Calvariensystem von Mäusen nachgewiesen werden kann.

Aufgrund dieser pharmakologischen Wirkungen können die Peptide der Formel I z.B. zur Behandlung von Krankheiten mit Osteopenien, wie Osteoporose, Osteoarthritis, Osteoarthrose oder Morbus Sudeck, verwendet werden, wobei Dosen von etwa 0,1 bis etwa 10 mg/Tag an einen Warmblüter von etwa 70 kg Körpergewicht verabreicht werden.

Somit betrifft die Erfindung auch eine Methode zur Behandlung von Krankheiten mit Osteopenien bei Warmblütern, dadurch gekennzeichnet, dass man an einen derartig erkrankten Warmblüter eine wirksame Dosis eines Peptids der Formel I oder eines pharmazeutisch verwendbaren Salzes davon verabreicht.

Ferner können die Peptide der Formel I zur Herstellung von diagnostischen Antikörpern zum Nachweis von medullären Schilddrüsenkarzinomen verwendet werden.

Die Erfindung betrifft ebenfalls pharmazeutische Präparate, die als Wirkstoff eine wirksame Dosis, insbesondere eine zur Behandlung der obengenannten Krankheiten wirksame Dosis, einer der erfindungsgemässen Verbindungen oder eines Salzes davon, vorzugsweise zusammen mit einem Trägermaterial, dessen Anteil üblicherweise mehr als 50 Gewichtsprozent beträgt, enthalten. Es handelt sich insbesondere um Präparate, die sich zur intranasalen oder parenteralen, wie subkutanen, intramuskulären oder intravenösen Verwendung an Warmblütern, wie am Menschen eignen, wobei die Dosierung des Wirkstoffes von der Art, dem Körpergewicht, Alter und dem individuellen Zustand, der zu behandelnden Krankheit, sowie von der Applikationsweise abhängt.

Die neuen pharmazeutischen Präparate zur parenteralen Applikation enthalten in gebrauchsfertiger Form von etwa 0,01 bis etwa 10 Gewichtsprozent, vorzugsweise von etwa 0,05 bis etwa 1 Gewichtsprozent des Wirkstoffes. Die erfindungsgemässen pharmazeutischen Präparate können z.B. in Dosiseinheitsform, wie Ampullen, vorliegen.

Vorzugsweise verwendet man Lösungen des Wirkstoffes, daneben auch Suspensionen, insbesondere isotonische wässrige Lösungen oder Suspensionen, wobei diese, z.B. bei lyophilisierten Präparaten, welche die Wirksubstanz allein oder zusammen mit einem Trägermaterial, z.B. Mannit, enthalten, vor Gebrauch hergestellt werden können. Zur intranasalen Anwendung besonders geeignet sind Suspensionen in Oel. Die pharmazeutischen Präparate können sterilisiert sein und können Hilfsstoffe, z.B. Konservier-, Stabilisier-, Netz und/oder Emulgiermittel, Löslichkeitsvermittler, Salze zur Regulierung des osmotischen Druckes und/oder Puffer, ferner viskositätserhöhende Stoffe, wie Natrium-Carboxymethylcellulose, Carboxymethylcellulose, Dextran, Polyvinylpyrrolidon oder Gelatine, enthalten. Sie können in an sich bekannter Weise, z.B. mittels konventioneller Lösungs- oder Lyophilisierungsverfahren, hergestellt werden.

Suspensionen in Oel können als ölige Komponente die für Injektionszwecke gebräuchlichen pflanzlichen, synthetischen oder halbsynthetischen Oele enthalten. Als solche sind insbesondere flüssige Fettsäureester zu nennen, die als Säurekomponente eine langkettige Fettsäure mit etwa 8 bis etwa 22 Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren, wie z.B. Oelsäure, Elaidinsäure, Erucasäure, Brasidinsäure oder Linolsäure, aufweisen. Die Alkoholkomponente enthält üblicherweise bis und mit 7 Kohlenstoffatome und stellt einen ein- oder mehrwertigen Alkohol, z.B. Methanol, Ethanol, Propanol, Butanol oder Pentanol oder deren Isomere, vor allem aber Glycol oder Glyzerin dar. Als Fettsäureester sind daher beispielsweise zu nennen: Ethyloleat, Isopropylmyristat, Isopropylpalmitat, Polyoxyethylenglyzerintrioleat (z B. "Labrafil M 2735" der Firma Gattefossé, Paris), Triglyzeride gesättiger Fettsäuren der Kettenlänge $C_8$ bis $C_{12}$ (z B. "Miglyol 812" der Firma Chemische Werke, Witten/Ruhr, Deutschland), ferner pflanzliche Oele, wie Baumwollsaatöl, Mandelöl, Olivenöl, Ricinusöl, Sesamöl, Sojabohnenöl oder Erdnussöl.

Die Herstellung der Injektionspräparate erfolgt in üblicher Weise unter antimikrobiellen Bedingungen, ebenso das Abfüllen in Ampullen oder Vials sowie das Verschliessen der Behälter.

Die Erfindung umfasst ebenfalls die Verwendung der Verbindungen der Formel I als pharmakologische Wirkstoffe bzw. zur Herstellung von pharmazeutischen Präparaten, in erster Linie zur Behandlung von Erkrankungen mit Osteopenien, wobei man vorzugsweise tägliche Dosen von etwa 0,1 bis etwa 10 mg verwendet.

Vorstehend mit "nieder" bezeichnete organische Reste und Verbindungen enthalten bis und mit 7, vorzugsweise bis und mit 4 Kohlenstoffatome.

Die nachfolgenden Beispiele illustrieren die Erfindung. Temperaturen sind in Celsiusgraden angegeben.

Beispiel 1:

1.1. Das nach dem unten beschriebenen Verfahren erhältliche Fmoc-Peptid/Harz-Ausgangsmaterial wird in einer vollautomatischen Peptidsyntheseapparatur folgenden Reaktions- und Waschoperationen unterworfen, wobei jeweils etwa 30 ml Waschflüssigkeit verwendet werden und das Reaktionsgefäss regelmässig geschüttelt wird; die Reaktionsstufen werden, sofern nicht anders angegeben, bei Raumtemperatur durchgeführt:

- einmaliges Waschen während 0,8 Minuten des Fmoc-Peptid/Harz-Ausgangsmaterials mit Isopropanol; .
- dreimaliges Waschen während je 0,4 Minuten mit unter vermindertem Druck entgastem Dimethylacetamid (Dimethylamin-frei);
- achtmaliges Behandeln während je 1,8 Minuten mit einer 20%igen Lösung von Piperidin in Dimethylacetamid (Abspaltung der Fmoc-Schutzgruppe);
- zweimaliges Waschen während je 0,4 Minuten mit unter vermindertem Druck entgastem Dimethylacetamid (Dimethylamin-frei);
- einmaliges Waschen während 0,8 Minuten mit Isopropanol;
- dreimaliges Waschen während je 0,4 Minuten mit unter vermindertem Druck entgastem Dimethylacetamid (Dimethylamin-frei);
- dreimaliges Waschen während je 0,4 Minuten mit destilliertem 1-Methyl-2-piperidon;
- Zugabe des zwischenzeitlich bereitgestellten ersten Kupplungsreagens, das wie folgt erhalten wird: 2,7 mMol Fmoc-L-alanin werden in 6,75 ml eines 0,4 molaren Gemisches von 1-Hydroxy-1H-benztriazol in 1-Methyl-2-piperidon gelöst und mit 6,48 ml einer 0,5 molaren Lösung von Diisopropylcarbodiimid in 1-Methyl-2-piperidon versetzt. Das Reaktionsgemisch wird während etwa 40 Minuten bei Raumtemperatur gehalten und dann in der vorliegenden Form verwendet. Die Kupplungsreaktion selber dauert 40 Minuten, das Reaktionsgemisch wird bei 50° gehalten;
- dreimaliges Waschen während je 0,4 Minuten mit destilliertem 1-Methyl-2-piperidon;
- Zugabe des zwischenzeitlich bereitgestellten zweiten Kupplungsreagens, das die gleiche Zusammensetzung hat, wie das erste, und in gleicher Weise hergestellt wird; die Kupplungsreaktion wird bei 50° durchgeführt und dauert 30 Minuten (mit der zweiten Kupplungsreaktion kann eine nahezu vollständige Umsetzung erzielt werden);
- einmaliges Waschen während 0,4 Minuten mit unter vermindertem Druck entgastem Dimethylacetamid (Dimethylamin-frei);
- einmaliges Behandeln während 4,5 Minuten mit etwa 30 ml eines 1:1:8-Gemisches (v/v/v) von Essigsäureanhydrid, Pyridin und Dimethylacetamid (zur Acetylierung von noch freien Aminogruppen, wobei die N-acetylierten Verbindungen im nachfolgenden Aufarbeitungsverfahren entfernt werden);
- dreimaliges Waschen während je 0,4 Minuten mit unter vermindertem Druck entgastem Dimethylacetamid (Dimethylamin-frei);
- einmaliges Waschen während 0,8 Minuten mit Isopropanol;
- dreimaliges Waschen während 0,4 Minuten mit unter vermindertem Druck entgastem Dimethylacetamid (Dimethylamin-frei);
- achtmaliges Behandeln während je 1,8 Minuten mit einer 20%igen Lösung von Piperidin in Dimethylacetamid (Abspaltung der Fmoc-Schutzgruppe);
- zweimaliges Waschen während je 0,4 Minuten mit unter vermindertem Druck entgastem Dimethylacetamid (Dimethylamin-frei);

-einmaliges Waschen während 0,8 Minuten mit Isopropanol;

-dreimaliges Waschen während je 0,4 Minuten mit unter vermindertem Druck entgastem Dimethylacetamid (Dimethylamin-frei); und

-dreimaliges Waschen während je 0,4 Minuten mit destilliertem 1-Methyl-2-piperidon.

Nach der Durchführung dieser Verfahrensschritte wird die so erhaltene Peptid/Harz-Verbindung fünfmal mit Isopropanol gewaschen und unter vermindertem Druck (Hochvakuum) getrocknet.

1.2. Zur Abspaltung despolymeren Trägers, sowie gegebenenfalls eines Teils der säurelabilen Schutzgruppen werden 1,1 g der nach dem obigen Verfahren erhältlichen Peptid/Harz-Verbindung (etwa 0,1 mMol) in 6 ml eines 98:2-Gemisches (v/v) von Trifluoressigsäure (95%ig) und Ethandithiol während 5 Minuten bei Raumtemperatur geschüttelt, dann filtriert, und der Filterrückstand nochmals mit der gleichen Menge des Trifluoressigsäure-Ethandithiol-Gemisches während 5 Minuten behandelt, dann filtriert. Anschliessend wird der Filterrückstand zweimal mit je 6 ml 1,2-Dichlorethan und zweimal mit je 6 ml Trifluorethanol gewaschen. Die vereinigten Filtrate und Waschflüssigkeiten werden bei Raumtemperatur unter vermindertem Druck auf ein Volumen von etwa 5 ml konzentriert, und das rohe Peptid durch Zugabe von 25 ml eines 1:1-Gemisches (v/v) von Diisopropylether und Petrolether (niedrigsiedend) gefällt. Der Niederschlag wird abfiltriert und unter vermindertem Druck (Hochdruck) getrocknet.

Die vollständige Abspaltung der Schutzgruppen kann durch Lösen des Rohpeptids in 5 ml eines 98:2-Gemisches (v/v) von Trifluoressigsäure (95 %) und Ethandithiol erzielt werden; die Lösung wird während 60 Minuten bei Raumtemperatur gehalten, und das Peptid wiederum durch Zugabe von 25 ml des 1:1-Gemisches (v/v) von Diisopropylether und Petrolether (niedrigsiedend) gefällt; diese Behandlung wird nochmals wiederholt, wobei die Reaktionszeit 50 Minuten beträgt. Der Niederschlag wird in 20 ml 10%iger wässriger Essigsäure gelöst und lyophilisiert; man erhält das Rohpeptid in Form eines weissen Pulvers.

1.3. Zur Reduktion von gegebenenfalls vorhandenem Methioninsulfoxid wird 0,1 g des erhaltenen Rohpeptids in 1 ml Trifluoressigsäure (90%ig) gelöst, eine Lösung von 0,015 g Ammoniumjodid in 0,1 ml Wasser zugegeben und nach 10 Minuten das Peptid bei Raumtemperatur durch Zugabe von 6 ml eines 1:1-Gemisches (v/v) von Diisopropylether und Petrolether (niedrigsiedend) gefällt. Das so erhaltene rötliche Oel wird in 2 ml 10%iger wässriger Essigsäure gelöst und bis zur vollständigen Entfärbung portionenweise mit total 0,01 g Ascorbinsäure versetzt. Die Lösung wird einer Gelfiltration unterworfen, wobei eine Säule aus einem Polyacrylamidprodukt mit einem Trennbereich von 1000 bis 6000 (Biogel-P6-säule der Firma Bio-Rad) verwendet wird. Es wird mit 10%iger wässriger Essigsäure eluiert, wobei das gewünschte Rohpeptid als erste Fraktion nach Auswaschen mit etwa 40 ml erhalten wird; durch Lyophilisieren wird das Produkt in Form eines weissen Pulvers erhalten.

265 mg Biogel filtriertes Produkt werden in einer Gegenstromverteilungsapparatur mit 3 ml Phasenvolumen im System tert. Amylalkohol-Essigsäure-Wasser (4:2:5; v:v) ab den Gefässen 3-5 über 1900 Stufen verteilt. 2 Randfraktionen aus den Gefässen 33-39 und den Gefässen 81-90 werden entnommen. Die hochgereinigte Hauptfraktion aus den Gefässen 40-80 ergibt nach Konzentrieren im Vakuum und Lyophilisieren das Material, welches weiterverarbeitet wird.

1.4. Zur Reinigung wird 0,065 g des so erhältlichen Rohpeptids in 6,5 ml 20%iger wässriger Essigsäure gelöst und in zwei Portionen (2,2 ml und 4,3 ml) unter folgenden Bedingungen der Hochdruckflüssigkeitschromatographie unterworfen: die Säule mit einer Dimension von 20x250 mm besteht aus Silicagel, belegt mit aliphatischen Ketten (Nucleosil 7C18 der Firma Macherey-Nagel, Düren, Bundesrepublik Deutschland), als Eluierungsmittel (A) wird 0,1%ige wässrige Trifluoressigsäure, und als Eluierungsmittel (B) eine 0,1%ige Lösung von Trifluoressigsäure in Acetonitril verwendet. Der lineare Gradient beträgt 15 % B → 45 % B in 50 Minuten, 45 % B → 60 % B in 10 Minuten, 60 % B → 100 % B in 10 Minuten, die Durchlaufgeschwindigkeit 18 ml/Min., und die Detektion im UV-Licht 215 nm. Die Hauptfraktion mit einer Retentionszeit von etwa 45 Minuten wird gesammelt, unter vermindertem Druck (Hochvakuum) konzentriert und dann lyophilisiert. Man erhält so das Peptid PCATFP der Formel

```
H-Ala-Pro-Phe-Arg-Ser-Ala-Leu-Glu-Ser-Ser

    Pro-Ala-Asp-Pro-Ala-Thr-Leu-Ser-Glu-Asp

    Glu-Ala-Arg-Leu-Leu-Leu-Ala-Ala-Leu-Val

    Gln-Asp-Tyr-Val-Gln-Met-Lys-Ala-Ser-Glu

    Leu-Glu-Gln-Glu-Gln-Glu-Arg-Glu-Gly-Ser

    Ser-Leu-Asp-Ser-Pro-Arg-Ser-OH
```

in Form eines weissen Pulvers.

Die Hochdruckflüssigkeitschromatographie (Säule: Silicagel, belegt mit aliphatischen Ketten (Nucleosil 5C18 der Firma Macherey-Nagel Düren, Bundesrepublik Deutschland); Dimension: 4,6 x 250 mm, Eluierflüssigkeit A: 0,1%ige wässrige Trifluoressigsäure, und Eluierflüssigkeit B: 0,1%ige Lösung von Trifluoressigsäure in Acetonitril; linearer Gradient 0 % B → 90 % B in 60 Minuten; Durchlaufgeschwindigkeit: 1 ml/Min; Detektion im UV-Licht: 215 nm) für das so erhältliche einheitliche Produkt ergibt eine Retentionszeit von 37,8 Minuten. Die Plasmadesorptions-Massenspektrometrie (257 Cf) ergibt das entsprechende Molekulargewicht.

FAB-MS: MH+ 6220 (berechnetes Molekulargewicht: 6220), isoelektrischer Punkt: 4,2 (berechnet: 4,04).

Das Ausgangsmaterial kann in folgender Weise erhalten werden: Ausgehend von sogenanntem Fmoc-Ser(But)-p-benzyloxybenzylesterpolystyrolharz (1 % vernetzt) der Firma Novabiochem, Läufelfingen, Schweiz), worin die Carboxylgruppe des L-Serins, dessen Aminogruppe durch 9-Fluorenyl-methoxycarbonyl (Fmoc) und dessen Hydroxygruppe durch tert.-Butyl (But) geschützt sind, mit 4-Methoxy-benzylalkohol verestert ist, wobei das Kohlenstoffatom der Methoxygruppe mit einem aromatischen Ring des zu 1 % mit Divinylbenzol vernetzten Polystyrolharzes, das gleichzeitig als Trägermaterial dient, verbunden ist, wird der Aufbau des Peptidmoleküls nach der Merrifield-Synthese durchgeführt. Dabei wird eine vollautomatische Peptidsyntheseapparatur verwendet, die sich zur alternierenden Abspaltung der Aminoschutzgruppen, im vorliegenden Fall der Fmoc-Gruppe, und zur Ankupplung der Fmoc-Aminosäurederivate ohne Isolierung der jeweils erhältlichen Peptid/Harz-Zwischenprodukte eignet. Im ersten Schritt wird dabei Fmoc-Arg(Pmc)-OH (Pmc: 2,2,5,7,8-Pentamethyl-chroman-6-sulfonyl) mit dem Ser(But)/Harz-Ausgangsmaterial gekuppelt, und dann die weiteren Fmoc-Aminosäuren stufenweise in der Reihenfolge Fmoc-Pro-OH, Fmoc-Ser(But)-OH, Fmoc-Asp(OBut)-OH (die nicht an der Reaktion teilnehmende zweite Carboxylgruppe liegt in Form des tert.-Butylesters vor), Fmoc-Leu, Fmoc-Ser(But)-OH, Fmoc-Ser(But)-OH, Fmoc-Gly-OH, Fmoc-Glu(OBut)-OH, Fmoc-Arg(Pmc)-OH, Fmoc-Glu(OBut)-OH, Fmoc-Gln-0H, Fmoc-Glu(OBut)-OH, Fmoc-Gln-OH, Fmoc-Glu(OBut)-OH, Fmoc-Leu-OH, Fmoc-Glu(OBut)-OH, Fmoc-Ser(But)-OH, Fmoc-Ala-OH, Fmoc-Lys(Boc)-OH, Fmoc-Met-OH, Fmoc-Gln-OH, Fmoc-Val-OH, Fmoc-Tyr(But)-OH, Fmoc-Asp(OBut)-0H, Fmoc-Gln-OH, Fmoc-Val-OH, Fmoc-Leu-OH, Fmoc-Ala-OH, Fmoc-Ala-OH, Fmoc-Leu-OH, Fmoc-Leu-OH, Fmoc-Leu-OH, Fmoc-Arg(Pmc)-OH, Fmoc-Ala-OH, Fmoc-Glu(OBut)-OH, Fmoc-Asp(OBut)-OH, Fmoc-Glu(OBut)-OH, Fmoc-Ser(But)-OH, Fmoc-Leu-OH, Fmoc-Thr(But)-OH, Fmoc-Ala-OH, Fmoc-Pro-OH, Fmoc-Asp(OBut)-OH, Fmoc-Ala-OH, Fmoc-Pro-OH, Fmoc-Ser(But)-OH, Fmoc-Ser(But)-OH, Fmoc-Glu(OBut)-OH, Fmoc-Leu-OH, Fmoc-Leu-OH, Fmoc-Ala-OH, Fmoc-Ser(But)-OH, Fmoc-Arg(Pmc)-0H, Fmoc-Phe-OH und Fmoc-Pro-OH an das jeweils erhältliche Peptid/Harz-Zwischenprodukt gekuppelt. Die einzelnen Stufen werden nach folgendem Schema durchgeführt, wobei jeweils etwa 30 ml der Waschflüssigkeiten verwendet und die einzelnen Operationen, falls nicht anders angegeben, bei Raumtemperatur durchgeführt werden, und das Reaktionsgemisch regelmässig geschüttelt wird:

Ausgehend von 2 g des oben beschriebenen Fmoc-Ser(But)/Harz-Ausgangsmaterials werden die folgenden, sich für jede Stufe jeweils repetierenden Verfahrungsschritte durchgeführt:
- einmaliges Waschen während 0,8 Minuten mit Isopropanol;
- dreimaliges Waschen während je 0,4 Minuten mit unter vermindertem Druck entgastem Dimethylacetamid (Dimethylamin-frei);
- achtmaliges Behandeln während je 1,8 Minuten mit einer 20%igen Lösung von Piperidin in Dimethylacetamid (Abspaltung der Fmoc-Schutzgruppe);
- zweimaliges Waschen während je 0,4 Minuten mit unter vermindertem Druck entgastem Dimethylacetamid (Dimethylamin-frei);
- einmaliges Waschen während 0,8 Minuten mit Isopropanol;
- dreimaliges Waschen während je 0,4 Minuten mit unter vermindertem Druck entgastem Dimethylacetamid (Dimethylamin-frei);
- dreimaliges Waschen während je 0,4 Minuten mit destilliertem 1-Methyl-2-piperidon;
- Zugabe des zwischenzeitlich bereitgestellten ersten Kupplungsreagens, das wie folgt erhalten wird: 2,7 mMol der jeweiligen Fmoc-L-Aminosäure werden in 6,75 ml eines 0,4 molaren Gemisch von 1-Hydroxy-1H-benztriazol in 1-Methyl-2-piperidon gelöst und mit 6,48 ml einer 0,5 molaren Lösung von Diisopropylcarbodiimid in 1-Methyl-2-piperidon versetzt. Das Reaktionsgemisch wird während etwa 40 Minuten bei Raumtemperatur und unter Rühren gehalten und dann in der vorliegenden Form verwendet. Die Kupplungsreaktion selber dauert 40 Minuten, wobei das Reaktionsgemisch bei 50° gehalten wird;
- dreimaliges Waschen während je 0,4 Minuten mit destilliertem 1-Methyl-2-piperidon;
- Zugabe des zwischenzeitlich bereitgestellten zweiten Kupplungsreagens, das die gleiche Zusammensetzung hat, wie das erste, und in gleicher Weise hergestellt wird; die Kupplungsreaktion wird bei 50° durchgeführt und dauert 30 Minuten (mit der zweiten Kupplungsreaktion kann eine nahezu vollständige Umsetzung erzielt werden);
- einmaliges Waschen während 0,4 Minuten mit unter vermindertem. Druck entgastem Dimethylacetamid (Dimethylamin-frei);
- einmaliges Behandeln während 4,5 Minuten mit etwa 30 ml eines 1:1:8-Gemisches (v/v/v) von Essigsäureanhydrid, Pyridin und Dimethylacetamid (zur Acetylierung von noch freien Aminogruppen in der wachsenden Peptidkette);
- dreimaliges Waschen während je 0,4 Minuten mit unter vermindertem Druck entgastem Dimethylacetamid (Dimethylamin-frei);
- einmaliges Waschen während 0,8 Minuten mit Isopropanol, und
- dreimaliges Waschen während 0,4 Minuten mit unter vermindertem Druck entgastem Dimethylacetamid (Dimethylamin-frei).

Man erhält so das als Ausgangsmaterial im obigen Beispiel verwendete Fmoc-Peptid/Harz Zwischenprodukt der Formel

Fmoc-Pro-Phe-Arg(Pmc)-Ser(But)-Ala-Leu-Glu(OBut)-Ser(But)-Ser(But-)Pro-Ala-Asp(OBut)-Pro-Ala-Thr(But)-Leu-Ser(But)-Glu(OBut)-Asp(OBut)-Glu(OBut)-Ala-Arg(Pmc)-Leu-Leu-Leu-Ala-Ala-Leu-Val-Gln-
Asp(OBut)-Tyr(But)-Val-Gln-Met-Lys(Boc)-Ala-Se        r(But)-Glu(OBut)-Leu-Glu(OBut)-Gln-Glu(OBut)-Gln-
Glu(OBut)-Arg(Pmc)-Glu(OBut)-Gly-Ser(But)-Ser(But)-Leu-Asp(OBut)-Ser(But)-Pro-
Arg(Pmc)-Ser(But)-Harz

wobei unter "Harz" der die Carboxylgruppe veresternde Polystyrol(1 % mit Divinylbenzol vernetzt)-methoxy-4-phenylmethoxy-Rest zu verstehen ist.

Beispiel 2: Sterile Trockensubstanz zur Injektion

Man löst 0,1 mg des PCATFP-Peptids (Beispiel 1) in 1 ml einer wässrigen Lösung zusammen mit 20 mg Mannit. Die Lösung wird sterilfiltriert und unter aseptischen Bedingungen in eine 2 ml Ampulle abgefüllt, tiefgekühlt und lyophilisiert. Vor dem Gebrauch wird das Lyophilisat in 1 ml destilliertem Wasser oder 1 ml physiologischer Salzlösung gelöst, und die Lösung z.B. intramuskulär oder intravenös verwendet.

Beispiel 3:

In analoger Weise wie im Beispiel 1, jedoch durch Acetylierung der nach Abspaltung der Fmoc-Schutzgruppe erhaltenen freien Aminogruppe des terminalen Alanins mit Essigsäureanhydrid wie beschrieben in Beispiel 1, erhält man

```
CH₃-CO-Ala-Pro-Phe-Arg-Ser-Ala-Leu-Glu-Ser-Ser

        Pro-Ala-Asp-Pro-Ala-Thr-Leu-Ser-Glu-Asp

        Glu-Ala-Arg-Leu-Leu-Leu-Ala-Ala-Leu-Val

        Gln-Asp-Tyr-Val-Gln-Met-Lys-Ala-Ser-Glu

        Leu-Glu-Gln-Glu-Gln-Glu-Arg-Glu-Gly-Ser

        Ser-Leu-Asp-Ser-Pro-Arg-Ser-OH
```

Beispiel 4:

In analoger Weise wie im Beispiel 1, jedoch ausgehend von einem wie unten beschrieben hergestellten Syntheseharz, unter Anknüpfung der der Struktur des gewünschten Endprodukts entsprechenden geschützten Aminosäuren, wobei man aber Fmoc-Gln(Trityl)-OH anstelle von Fmoc-Gln-OH verwendet, sowie unter schlussendlicher Abspaltung aller restlichen Schutzgruppen mit Trifluoressigsäure-Wasser-1,2-Ethandithiol (90:5:5) während 40 Minuten bei Raumtemperatur, erhält man

```
H-Ala-Pro-Phe-Arg-Ser-Ala-Leu-Glu-Ser-Ser-

    Pro-Ala-Asp-Pro-Ala-Thr-Leu-Ser-Glu-Asp-

    Glu-Ala-Arg-Leu-Leu-Leu-Ala-Ala-Leu-Val-

    Gln-Asp-Tyr-Val-Gln-Met-Lys-Ala-Ser-Glu-

    Leu-Glu-Gln-Glu-Gln-Glu-Arg-Glu-Gly-Ser-

    Arg-Ile-Ile-Ala-Gln-OH
```

Die Ausgangsprodukte erhält man folgendermassen:

Fmoc-Gln(Trt)-OH

Eine Lösung von 4g des Halbhydrates von H-Gln(Trt)-OH (10 mMol) in 20 ml Tetrahydrofuran und 10 ml einnormaler wässriger Natriumhydroxidlösung wird unter gutem Rühren mit 3,4 g 9-Fluorenylmethyl-succinimidinylcarbonat (10 mMol) versetzt und gleichzeitig 1,4 ml Triethylamin so zugetropft, dass der pH-Wert etwa 8,5 beträgt. Nach 20 Minuten wird im Eisbad gekühlt, mit Essigsäureethylester überschichtet und mit 25 ml. einmolarer wässriger Kaliumhydrogensulfatlösung bis zu einem pH-Wert von 2-2,5 angesäuert. Die organische Phase wird mit Wasser neutral gewaschen, über Natriumsulfat getrocknet und unter vermindertem Druck auf 30 g eingeengt. Das Produkt wird durch portionenweise Zugabe von 35 ml Diisopropylether zum Kristallisieren gebracht. Nach längerem Stehen in der Kälte wird das kristalline Material abfiltriert und einmal in einem 1:4-Gemisch von Essigsäureethylester und Diisopropylether und dreimal mit Diisopropylether gewaschen. Das Fmoc-Gln(Trt)-OH enthält auch nach längerem Trocknen im Hochvakuum bei 60° noch 0,5 Mol Diisopropylether, Smp. ab 125° (langsames Schmelzen unter Abgabe von Diisopropylether).

### Fmoc-Gln(Trt)-OH

Ein Gemisch von 7,4g Fmoc-Gln-OH (20 mMol) und 10,4 g Triphenylmethanol (40 mMol) in 300 ml Essigsäure wird 10 Minuten bei 100° gerührt. Nach Zugabe von 0,1 ml konz. Schwefelsäure wird noch weitere 15 Minuten bei 100° gerührt, wobei eine klare, gelbe Lösung entsteht. Diese wird unter vermindertem Druck bei 60° eingedampft, und der Rückstand noch 10 Minuten unter vermindertem Druck getrocknet. Der feste Rückstand wird in 60 ml Essigsäure bei 60° gelöst, während 20 Minuten bei 60° belassen, dann mit 2 ml Essigsäureanhydrid (20 mMol) versetzt und nach 1 Stunde bei 60° unter vermindertem Druck eingedampft. Der ölige Rückstand wird während 10 Minuten bei 60° getrocknet, dann in 100 ml Essigsäure gelöst und in 500 ml eiskaltes Wasser eingetropft. Der Niederschlag wird abfiltriert, gut mit Wasser gewaschen und in Essigsäureethylester gelöst; die wässrige Phase wird abgetrennt, und die organische Phase mit einer gesättigten wässrigen Natriumchloridlösung gewaschen. Nach dem Trocknen über Natriumsulfat wird die organische Lösung zur Trockne eingedampft, der Rückstand wird in 50 ml 1,2-Dichlorethan gelöst und auf 100 g Silikagel gegeben; eluiert wird mit 1,2-Dichlorethan und 1,2-Dichlorethan, enthaltend 2 %,dann 4 % Methanol. Die das Fmoc-Gln(Trt)-OH enthaltenden Fraktionen werden unter vermindertem Druck eingedampft, und das Produkt wird aus einem Gemisch von Essigsäureethylester und Diisopropylether kristallisiert, Smp. ab 125°.

### Syntheseharz

Zu einer Suspension von 10 g sogenanntem 4-Benzyloxybenzylalkoholpolystyrol (0,78 mMol OH/g; 1 % mit Divinylbenzol quervernetzt; die Phenylgruppen des Polystyrols sind in 4-Stellung durch 4-Hydroxymethylphenyloxymethyl substituiert; Novabiochem), 15,6 mMol Fmoc-Gln(Trt)-OH und 3,1 ml (38,5 mMol) Pyridin in 60 ml Dimethylacetamid werden 3,35 ml (23,4 mMol) 2,6-Dichlor-benzoylchlorid (Fluka) in 4 Portionen innerhalb von 5 Stunden zugegeben und 24 Stunden gerührt. Das Harz wird abfiltriert und sehr gut mit Methanol und 1,2-Dichlor-ethan ausgewaschen. Das Harz weist eine Beladung von 0,48 mMol/g auf (photometrische Fmoc Bestimmung). Die restlichen OH-Gruppen werden vor Beginn der Peptidsynthese 2 Stunden lang mit Acetanhydrid-Pyridin-Dimethylacetamid 1:1:8 acetyliert.

### Beispiel 5:

In analoger Weise wie im Beispiel 4, jedoch durch Acetylierung der nach Abspaltung der Fmoc-Schutzgruppe erhaltenen freien Aminogruppe des terminalen Alanins mit Essigsäureanhydrid wie beschrieben in Beispiel 1, erhält man

$$CH_3-CO-Ala-Pro-Phe-Arg-Ser-Ala-Leu-Glu-Ser-Ser-$$
$$Pro-Ala-Asp-Pro-Ala-Thr-Leu-Ser-Glu-Asp-$$
$$Glu-Ala-Arg-Leu-Leu-Leu-Ala-Ala-Leu-Val-$$
$$Gln-Asp-Tyr-Val-Gln-Met-Lys-Ala-Ser-Glu-$$
$$Leu-Glu-Gln-Glu-Gln-Glu-Arg-Glu-Gly-Ser-$$
$$Arg-Ile-Ile-Ala-Gln-OH \qquad \cdot$$

**Patentansprüche**

1. Peptide der Formel I,

$$R-Ala-Pro-Phe-Arg-Ser-Ala-Leu-Glu-Ser-Ser-$$
$$Pro-Ala-Asp-Pro-Ala-Thr-Leu-Ser-Glu-Asp-$$
$$Glu-Ala-Arg-Leu-Leu-Leu-Ala-Ala-Leu-Val- \qquad (I)$$
$$Gln-Asp-Tyr-Val-Gln-Met-Lys-Ala-Ser-Glu-$$
$$Leu-Glu-Gln-Glu-Gln-Glu-Arg-Glu-Gly-Ser-X-OH$$

worin R Wasserstoff oder Acetyl und X die Aminosäuresequenz der Formel -Ser-Leu-Asp-Ser-Pro-Arg-Ser- (Ia) oder der Formel -Arg-Ile-Ile-Ala-Gln- (Ib) bedeutet, und Salze von solchen Verbindungen.

2. Peptide gemäss Anspruch 1 der Formel I, worin R Wasserstoff bedeutet und X die in Anspruch 1 gegebenen Bedeutungen hat, und Salze von solchen Verbindungen.

3. Peptid der Formel I gemäss Anspruch 2, worin X für die Aminosäuresequenz der Formel -Ser-Leu-Asp-Ser-Pro-Arg-Ser- (Ia) steht, und Salze von solchen Verbindungen.

4. Pharmazeutische Präparate, enthaltend eine der Verbindungen der Formel I gemäss einem der Ansprüche 1-3 oder ein pharmazeutisch verwendbares Salz davon zusammen mit pharmazeutischem Trägermaterial.

5. Eine Verbindung gemäss einem der Ansprüche 1-3 der Formel I oder ein pharmazeutisch verwendbares Salz davon zur Anwendung für die Behandlung des menschlichen und tierischen Körpers.

6. Verwendung von Verbindungen gemäss Ansprüchen 1-3 zur Herstellung von pharmazeutischen Präparaten zur Anwendung für die Behandlung von Erkrankungen mit Osteopenien.

7. Verfahren zur Herstellung von Peptiden der Formel I gemäss Anspruch 1 und von Salzen von solchen Verbindungen, dadurch gekennzeichnet, dass man

a) eine, in einer Verbindung mit der Aminosäuresequenz der Formel I vorhandene Amidbindung durch Umsetzen eines Fragments einer solchen Verbindung, das eine reaktionsfähige freie Carboxylgruppe aufweist, oder eines reaktionsfähigen Carbonsäurederivats davon mit dem komplementären Fragment einer solchen Verbindung, das eine freie Aminogruppe aufweist, oder mit einem reaktionsfähigen Derivat davon, wobei in den genannten Fragmenten freie funktionelle Gruppen mit Ausnahme der beiden an der Reaktion teilnehmenden Gruppen gegebenenfalls in geschützter Form vorliegen, bildet und gegebenenfalls vorhandene Schutzgruppen abspaltet, oder

b) Wirtszellen, die mit einem Expressionsvektor transformiert sind, der eine von einer Expressionskontrollsequenz regulierte, für die Aminosäuresequenz der Formel I kodierende DNA-Sequenz enthält, vermehrt, und eine Verbindung mit der Aminosäuresequenz der Formel I, falls notwendig, aus den Wirtszellen freisetzt und isoliert,

und wenn notwendig, in einer erfindungsgemäss erhältlichen Verbindung mit einer Sulfoxidgruppierung diese in die Thiogruppe überführt, und, wenn erwünscht, ein verfahrensgemäss erhältliches Salz in die freie Verbindung überführt und/oder eine verfahrensgemäss erhältliche freie Verbindung in ein Salz überführt.

8. Neue, für Peptide gemäss Anspruch 2 oder 3 kodierende DNAs.

9. Expressionsvektoren, enthaltend für Peptide gemäss Anspruch 2 oder 3 kodierende DNAs.

10. Mikroorganismen, enthaltend Vektoren gemäss Anspruch 9.

**Patentansprüche für folgende Vertragsstaaten: ES, GR**

1. Verfahren zur Herstellung eines Peptids der Formel I,

R-Ala-Pro-Phe-Arg-Ser-Ala-Leu-Glu-Ser-Ser-

    Pro-Ala-Asp-Pro-Ala-Thr-Leu-Ser-Glu-Asp-

    Glu-Ala-Arg-Leu-Leu-Leu-Ala-Ala-Leu-Val-        (I)

    Gln-Asp-Tyr-Val-Gln-Met-Lys-Ala-Ser-Glu-

    Leu-Glu-Gln-Glu-Gln-Glu-Arg-Glu-Gly-Ser-X-OH

worin R Wasserstoff oder Acetyl und X die Aminosäuresequenz der Formel -Ser-Leu-Asp-Ser-Pro-Arg-Ser- (Ia) oder der Formel -Arg-Ile-Ile-Ala-Gln- (Ib) bedeutet, oder eines Salzes einer solchen Verbindung, dadurch gekennzeichnet, dass man

a) eine, in einer Verbindung mit der Aminosäuresequenz der Formel I vorhandene Amidbindung durch Umsetzen eines Fragments einer solchen Verbindung, das eine reaktionsfähige freie Carboxylgruppe aufweist, oder eines reaktionsfähigen Carbonsäurederivats davon mit dem komplementären Fragment einer solchen Verbindung, das eine freie Aminogruppe aufweist, oder mit einem reaktionsfähigen Derivat davon, wobei in den genannten Fragmenten freie funktionelle Gruppen mit Ausnahme der beiden an der Reaktion teilnehmenden Gruppen gegebenenfalls in geschützter Form vorliegen, bildet und gegebenenfalls vorhandene Schutzgruppen abspaltet, oder

b) Wirtszellen, die mit einem Expressionsvektor transformiert sind, der eine von einer Expressionskontrollsequenz regulierte, für die Aminosäuresequenz der Formel I kodierende DNA-Sequenz enthält, vermehrt, und eine Verbindung mit der Aminosäuresequenz der Formel I, falls notwendig, aus den Wirtszellen freisetzt und isoliert,

und wenn notwendig, in einer erfindungsgemäss erhältlichen Verbindung mit einer Sulfoxidgruppierung diese in die Thiogruppe überführt, und, wenn erwünscht, ein verfahrensgemäss erhältliches Salz in die freie Verbindung überführt und/oder eine verfahrensgemäss erhältliche freie Verbindung in ein Salz überführt.

2. Verfahren gemäss Anspruch 1, dadurch gekennzeichnet, dass man die Ausgangsstoffe so wählt, dass man ein Peptid der Formel I, worin R Wasserstoff bedeutet und X die in Anspruch 1 gegebenen Bedeutungen hat, oder ein Salz einer solchen Verbindung herstellt.

3. Verfahren gemäss Anspruch 2, dadurch gekennzeichnet, dass man die Ausgangsstoffe so wählt, dass man ein Peptid der Formel I, worin X für die Aminosäuresequenz der Formel -Ser-Leu-Asp-Ser-Pro-Arg-Ser- (Ia) steht, oder ein Salz einer solchen Verbindung herstellt.